Europäisches Patentamt

European Patent Office

Office européen des brevets

(19)

(11) **EP 0 958 830 A1**

(12) **EUROPEAN PATENT APPLICATION**

(43) Date of publication:
24.11.1999 Bulletin 1999/47

(51) Int. Cl.$^6$: **A61K 38/17**, A61K 38/24

(21) Application number: 98201695.8

(22) Date of filing: 20.05.1998

(84) Designated Contracting States:
**AT BE CH CY DE DK ES FI FR GB GR IE IT LI LU
MC NL PT SE**
Designated Extension States:
**AL LT LV MK RO SI**

(71) Applicant:
**Erasmus Universiteit Rotterdam
3015 GE Rotterdam (NL)**

(72) Inventor:
**The designation of the inventor has not yet been
filed**

(74) Representative:
**Smulders, Theodorus A.H.J., Ir. et al
Vereenigde Octrooibureaux
Nieuwe Parklaan 97
2587 BN 's-Gravenhage (NL)**

(54) **Immunoregulator from pregnant mammals**

(57) The invention relates to the field of immunology, more specifically to the field of immune-mediated disorders such as allergies, auto-immune disease, transplantation-related disease or chronic inflammatory disease. The invention provides an immunoregulator comprising an active component having an apparent molecular weight of 58 to 15 kilodalton as determined in gel-permeation chromatography, said active component capable of stimulating splenocytes obtained from a non-obese diabetes (NOD) mouse, said stimulated splenocytes delaying the onset of diabetes in a NOD-severe-combined-immunodeficient mouse reconstituted with said splenocytes, or comprising an active component functionally related thereto, for treatment of immune-mediated disease.

EP 0 958 830 A1

## Description

The invention relates to the field of immunology, more specifically to the field of immune-mediated disorders such as allergies, auto-immune disease, transplantation-related disease or chronic inflammatory disease.

Recent advances in humoral and cellular immunology, molecular biology and pathology have influenced current thinking about auto-immunity being a component of immune-mediated disease. These advances have increased our understanding of the basic aspects of antibody, B-cell, and T-cell diversity, the generation of cellular and humoral immune responses and their interdependence, the mechanisms of (self)-tolerance induction and the means by which immunological reactivity develops against auto-antigenic constituents.

Since 1900, the central dogma of immunology has been that the immune system does not normally react to self. However, it as recently become apparent that auto-immune responses are not as rare as once thought and that not all auto-immune responses are harmful; some responses play a distinct role in mediating the immune response in general. For example, certain forms of auto-immune response such as recognition of cell surface anigens encoded by the major histocompatibility complex (MHC) and of anti-idiotypic responses against self idiotypes are important, indeed essential, for the diversification and normal functioning of the intact immune system.

Apparently, an intricate system of checks and balances is maintained between various subsets of cells (i.e. T-cells) of the immune system, thereby providing the individual with an immune system capable of coping with foreign invaders. In that sense, auto-immunity plays a regulating role in the immune system.

However, it is now also recognised that an abnormal auto-immune response is sometimes a primary cause and at other times a secondary contributor to many human and animal diseases. Types of auto-immune disease frequently overlap, and more than one auto-immune disorder tends to occur in the same individual, especially in those with auto-immune endocrinopathies. Auto-immune syndromes may be mediated with lymphoid hyperplasia, malignant lymphocytic or plasma cell proliferation and immunodeficiency disorders such as hypogammaglobulinaemie, selective Ig deficiencies and complement component deficiencies.

Auto-immune diseases, such systemic lupus erythematosus, diabetes, rheumatoid arthritis, auto-immune thromocytopenia, to name a few, are characterised by auto-immune responses, for example directed against widely distributed self-antigenic determinants, or directed against organ- or tissue specific antigens. Such disease may follow abnormal immune responses against only one antigenic target, ore against many self antigens. In many instances, it is not clear whether auto-immune responses are directed against unmodified self-antigens or self-antigens that have been modified (or resemble) any of numerous agents such as viruses, bacterial antigens and haptenic groups.

There is as yet no established unifying concept to explain the origin and pathogenesis of the various autoimmune disorders. Studies in experimental animals support the notion that auto-immune diseases may result from a wide spectrum of genetic and immunological abnormalities which differ from one individual to another and may express themselves early or late in life depending on the presence or absence of many superimposed exogenous (viruses, bacteria) or endogenous (hormones, cytokines, abnormal genes) accelerating factors.

It is evident that similar checks and balances that keep primary auto-immune disease at bay are also compromised in immune mediated disorders, such as allergy (asthma), chronic inflammatory disease (i.e rheumatic disease, Sjögrens syndrome, multiple sclerosis), transplantation-related immune responses (graft-versus-host-disease, post-transfusion thrombocytopenia), and many others wherein the responsible antigens (at least initially) may not be self-antigens but wherein the immune response to said antigen is in principle not wanted and detrimental to the individual.

In general, said immune mediated disorders are difficult to treat. Often, broad-acting medication is applied, such as treatment with corticosteroids or any other broad acting anti-inflammatory agent that in many aspects may be detrimental to a treated individual. In general there is a need for better and more specific possibilities to regulate the checks and balances of the immune system and treat immune mediated disorders.

The non-obese diabetic (NOD) mouse is a model for auto-immune disease, in this case insulin-dependent diabetes mellitus (IDDM) which main clinical feature is elevated blood glucose levels (hyperglycemia). Said elevated blood glucose level is caused by auto-immune destruction of insulin-producing β cells in the islets of Langerhans of the pancreas (Bach et al. 1991, Atkinson et al. 1994). This is accompanied by a massive cellular infiltration surrounding and penetrating the islets (insulitis) composed of a heterogeneous mixture of CD4+ and CD8+ T lymphocytes, B lymphocytes, macrophages and dendritic cells (O'Reilly et al. 1991)

In general, T lymphocytes play a pivotal role in initiating the immune mediated disease process (Sempe et al. 1991, Miyazaki et al. 1985, Harada et al. 1986, Makino et al. 1986).

The NOD mouse represents a model in which auto-immunity against beta-cells, is the primary event in the development of IDDM. Diabetogenesis is mediated through a multifactorial interaction between a unique MHC class II gene and multiple, unlinked, genetic loci, as in the human disease. Moreover, the NOD mouse demonstrates beautifully the critical interaction between heredity and environment, and between primary and secondary auto-immunity, its clinical manifestation is for example depending on various external conditions, most importantly of the micro-organism load of the environment in which the NOD mouse is housed.

As for auto-immunity demonstrable in NOD mice, most antigen-specific antibodies and T-cell responses are measured after these antigens were detected as self-antigens in diabetic patients. Understanding the role these auto-antigens play in NOD diabetes may further allow to distinguish between pathogenic auto-antigens and auto-immunity that is an epiphenomenon.

CD4+ T-cells can be separated into at least two major subsets Th1 and Th2. Activated Th1 cells secrete IFN-$\gamma$ and TNF-$\alpha$, while Th2 cells produce IL-4, IL-5 and IL-10. Th1 cells are critically involved in the generation of effective cellular immunity, whereas Th2 cells are instrumental in the generation of humoral and mucosal immunity and allergy, including the activation of eosinophils and mast cells and the production of IgE (Abbas et al. 1996). A number of studies have now correlated diabetes in mice and human with Th1 phenotype development (Liblau et al. 1995, Katz et al. 1995) . On the other hand, Th2 T cells are shown to be relatively innocuous. Some have even speculated that Th2 T cells in fact, may be protective. Katz et al. have shown that the ability of CD4+ T cells to transfer diabetes to naive recipients resided not with the antigen specificity recognised by the TCR per se, but with the phenotypic nature of the T cell response. Strongly polarised Th1 T cells transferred disease into NOD neonatal mice, while Th2 T cells did not, despite being activated and bearing the same TCR as the diabetogenic Th1 T cell population. Moreover, upon co-transfer, Th2 T cells could not ameliorate the Th1-induced diabetes, even when Th2 cells were co-transferred in 10-fold excess (Pakala et al. 1997).

These findings underwrite the postulated existence of cells regulating the balance between activated Th-sub-populations. Possible disturbances in this balance that are induced by altered reactivity of such regulatory T cell populations can cause immune-mediated diseases, which results in absence or over-production of certain critically important cytokines (O'Garra et al. 1997). These Th-sub-populations are potential targets for pharmacological regulation of immune responses.

The invention provides a pharmaceutical composition for treating an immune-mediated disorder such as an allergy, auto-immune disease, transplantation-related disease or chronic inflammatory disease and/or provides an immunoregulator, for example for stimulating or regulating lymphocyte action comprising an active component having an apparent molecular weight of 58 to 15 kilodalton as determined in gel-permeation chromatography, said active component capable of stimulating splenocytes obtained from a 20-week-old female non-obese diabetes (NOD) mouse, said stimulated splenocytes delaying the onset of diabetes in a NOD-severe-combined-immunodeficient (NOD.scid) mouse reconstituted at 8 weeks old with said splenocytes, or comprising an active component functionally related thereto.

In one embodiment, the invention provides an pharmaceutical composition or immunoregulator wherein said active component is capable of inhibiting gamma-interferon production or stimulating interleukine-4 production of splenocytes obtained from a 20-week-old female non-obese diabetes (NOD) mouse. Preferably, said composition or immunoregulator is obtainable from a pregnant mammal, preferably a human, for example obtainable from a pharmacological preparation prepared to contain (placental) gonadotropins such as pregnant mare serum gonadotropin (PMSG) found in serum of pregnant mares, or pregnant mouse uterus extract (PMUE) extracted from uteri of gravid mice or human chorionic gonadotropin (hCG or HCG) found in blood or urine of pregnant woman.

Clinical grade preparations of gonadotropins such as hCG and PMSG have since long been used to help treat reproductive failure in situations where follicular growth or stimulation of ovulation is desired. Said preparations are obtained from serum or urine, and often vary in degree of purification and relative activity, depending on initial concentration in serum or urine and depending on the various methods of preparation used. In a particular embodiment, the invention provides a immunoregulator comprising an active component obtainable from a mammalian CG preparation present in a fraction which elutes with an apparent molecular weight of 58 to 15 kilodalton as determined in gel-permeation chromatography, said active component capable of stimulating splenocytes obtained from a non-obese diabetes (NOD) mouse, or comprising an active component functionally related to said active compound, for example wherein said stimulated splenocytes are capable of delaying the onset of diabetes in a NOD-severe-combined-immunodeficient mouse reconstituted with said splenocytes.

The invention also provides an immunoregulator wherein said active component is capable of inhibiting gamma-interferon production obtained from a non-obese diabetes (NOD) mouse. The invention also provides an immunoregulator wherein said active component is capable of stimulating interleukine-4 production of splenocytes obtained from a non-obese diabetes (NOD) mouse.

Anecdotal observations and laboratory studies indicated previously that hCG might have an anti-Kaposi's sarcoma and anti-human-immunodeficiency-virus effect (Treatment Issues, July/August 1995, page 15. It has been observed that clinical grade hCG preparations have a direct apoptotic (cytotoxic) effect on Kaposi's sarcoma (KS) in vitro and in immunodeficient patients and mice and a prohematopoetic effect on immunodeficient patients (Lunardi-Iskandar et al., Nature 375, 64-68; Gill et al., New. Eng. J. Med. 335, 1261-1269, 1996), and a direct inhibitory antiviral effect on human and simian immunodeficiency virus (HIV and SIV) (Lunardi-Iskandar et al., Nature Med. 4, 428-434, 1998). Said cytotoxic and anti-viral effects have also been attributed to an unknown hCG mediated factor (HAF), present in clinical grade preparations of hCG. However, commercial hCG preparations (such as CG-10, Steris Profasi, Pregnyl, Choragon, Serono Profasi, APL), have various effects. Analysis of several of these, (AIDS, 11: 1333-1340, 1997) for example shows that only some (such as CG-10, Steris Profasi) are KS-killing whereas others (Pregnyl, Choragon, Serono Profasi) were not. Secondly, recombinant subunits of ($\alpha$ or $\beta$) hCG were killing but intact recombinant hCH not. It was also found that the killing effect was also seen with lymphocytes. Therapy of KS has recently been directed at using beta-hCG for its anti-tumour effect Eur. J. Med Res. 21: 155-158, 1997, and it was reported that the beta-core fragment isolated from urine had the highest apoptotic activity on KS cells (AIDS, 11: ,713-721, 1997).

The invention provides an immunoregulator or a pharmaceutical composition for treating an immune-mediated disorder obtainable from a hCG preparation or a fraction derived thereof. The effects of said immunoregulator include a stimulating effect on lymphocyte populations (such as found in peripheral lymphocytes, thymocytes or splenocytes), instead of cytotoxic or anti-viral effects. The invention provides a method for treating an immune-mediated-disorder comprising subjecting an animal to treatment with at least one immunoregulator obtainable from a pregnant mammal. Said treatment can be direct, for example treatment can comprise providing said individual with a pharmaceutical composition, such as a hCG or PMSG preparation, comprising an immunoregulator as provided by the invention. It is also possible to provide said pharmaceutical composition with a fraction or fractions derived from a pregnant animal by for example sampling urine or serum or placental (be it of maternal or fetal origin) or other tissue or cells and preparing said immunoregulator comprising said active component from said urine or serum or tissue or cells by fractionation techniques known in the art (for example by gelpermeation chromatograpy) and testing for its active component by stimulating a NOD mouse or its splenocytes as described.

The invention also provides a method for in vitro stimulation of lymphocytes and transferring said stimulated lymphocytes as a pharmaceutical composition to an animal for treating said animal for an immune mediated disorder. In a particular embodiment of the invention a pharmaceutical composition is provided comprising lymphocytes stimulated in vitro with an immunoregulator provided by the invention.

In a preferred embodiment of the invention, said disorder comprises diabetes, yet other immune mediated disorders, such as chronic inflammation, can also be treated. The invention provides a method of treatment for an animal, preferably wherein said animal is human.

In a particular embodiment, a method provided by the invention is further comprising regulating relative ratios and /or cytokine activity or cytokine expression or marker expression of lymphocyte subset-populations in said animal, such as subset-populations that comprise Th1 or Th2 cells, or Th3 or Th8 cells, or other effector or regulatory T-cell populations.

The invention also provides an immunoregulator for use in a method according to the invention, and use of said immunoregulator, preferably obtainable from a pregnant mammal, for the production of a pharmaceutical composition for the treatment of an immune-mediated-disorder, preferably selected from a group consisting of allergies, auto-immune disease, transplantation-related disease and chronic inflammatory disease. In a particular embodiment said immunoregulator comprises a clinical grade hCG or PMSG preparation or a fraction derived thereof. For example, the invention provides use of a hCG preparation, or a preparation functionally equivalent thereto, for the preparation of a pharmaceutical composition for the treatment of diabetes.

The invention is further explained in the experimental part without limiting the invention thereto.

Experimental part

The non-obese diabetic (NOD) mouse is a model for auto-immune disease, in this case insulin-dependent diabetes-mellitus (IDDM), which main clinical feature is elevated blood glucose levels (hyperglycemia) . The elevated blood glucose levels are caused by the immune-mediated destruction of insulin-producing $\beta$ cells in the islets of Langerhans of the pancreas (Bach et al. 1991, Atkinson et al. 1994) . This destruction is accompanied by a massive cellular infiltration surrounding and penetrating of the islets (insulitis) by a heterogeneous mixture composed of a CD4+ and CD8+ T lymphocytes, B lymphocytes, macrophages and dendritic cells (O'Reilly et al. 1991). The easiest and most reliable way to detect the onset of diabetes in these mice is to test for glucose levels in the blood.

The NOD mouse represents a model in which auto-immunity against beta-cells is the primary event in the development of IDDM. In general, T lymphocytes play a pivotal role in initiating the disease process (Sempe et al. 1991, Miyazaki et al. 1985, Harada et al. 1986, Makino et al. 1986). Diabetogenesis is mediated through a multifactorial interaction between a unique MHC class II gene and multiple, unlinked, genetic loci as in the human disease. Moreover, the NOD mouse demonstrates beautifully the critical interaction between heredity and environment. Differences between the cleanliness of the housing conditions illustrates how environmental factors can effect the action of diabetes-mediated genes (Elias et al. 1994).

As for the auto-immunity recorded in NOD mice, most antigen-specific antibodies and T-cell responses have been studied after these antigens were detected as self-antigens in diabetic patients. Understanding the role that these auto-antigens play in NOD diabetes may allow to distinguish between primary pathogenic auto-antigens and auto-immunity that is an epiphenomenon. Moreover, one should bear in mind that IDDM patients are genetically and pathogenically heterogeneous.

A typical longitudinal histological examination of the NOD pancreas demonstrates infiltrating cells surrounding the blood vessels at 3-4 weeks of age, but the islets are typically still clear at 6-7 weeks. Infiltrating cells than reach the islets, either surrounding them or accumulating at one pole. Between 10 and 12 weeks, the infiltrating cells penetrate into the islets and the islets become swollen with lymphocytes. As mentioned above, differences between the housing conditions and microbiological and environmental factors can effect the penetrance of diabetes-susceptible genes.

In our hands, typically between 14-17 weeks NOD mice become diabetic. However, this varies from lab to lab (average 14-19 weeks) (Elias et al. 1994).

CD4+ T-cells can be separated into at least two major subsets Th1 and Th2. Activated Th1 cells secrete IFN-$\gamma$ and TNF-$\alpha$, while Th2 cells produce IL-4, IL-5 and IL-10. Th1 cells are critically involved in the generation of effective cellular immunity, whereas Th2 cells are instrumental in the generation of humoral and mucosal immunity and allergy, including the activation of eosinophils and mast cells and the production of IgE (Abbas et al. 1996) . A number of studies have now correlated diabetes in mice and human with Th1 phenotype development (Liblau et al. 1995, Katz et al. 1995).

Th2 T cells are shown to be relatively innocuous. Some have even speculated that Th2 T cells in fact, may be protective. But Katz et al. have shown the ability of CD4+ T cells to transfer diabetes to naive recipients resided not with the antigen specificity recognised by the TCR, per se, but with the phenotypic nature of the T cell response. Strongly polarised Th1 T cells transferred disease into NOD neonatal mice, while Th2 T cells did not, despite being activated and bearing the same TCR as the diabetogenic Th1 T cell population. Moreover, upon co-transfer, Th2 T cells could not ameliorate Th1-induced diabetes, even when Th2 cells were co-transferred in 10-fold excess (Pakala et al. 1997).

Th1-polarized T cells can transfer disease in neonatal NOD mice, something Th2-polarized T cells fail to do, both Th1- and Th2-polarized T cells can transfer disease in NOD.scid mice and other immune-compromised recipients. Th2-mediated diabetes in NOD.scid recipients exhibited a longer pre-diabetic phase and a lowered overall incidence. Moreover, the diabetic lesion created by Th2 cells is unique and quite unlike the lesion found in spontaneously diabetic or Th1 T cell-induced diabetes in either neonates or NOD.scid mice (Pakala et al. 1997).

In addition, IFN-$\gamma$ correlates with diabetes (in NOD as well as in humans) and anti-IFN-$\gamma$ prevents disease; under disease IFN-$\gamma$+ cells are present in islets and antigen-specific Th1 clones accelerate the onset of diabetes (Pakala et al. 1997, O'Garra et al. 1997) . Furthermore, Th2 cells only induce insulitis in neonatal NOD, but have the capacity to induce diabetes in immuno-compromised NOD.scid; also, disease is inhibitable by anti-IL-10, but not by anti-IL-4 (Pakala et al. 1997). This suggests that non-Th2 type regulator T cells are present in normal mice, but these are absent in immunodeficient mice. These results stress the existence of cells regulating the balance between activated Th-sub-populations. Possible disturbances in this balance induced by altered reactivity of such regulatory T cell populations can cause immune-mediated diseases, which results in absence or over-production of certain critically important cytokines (O'Garra et al. 1997).

Recently, Gallo et. al. reported anti-Kaposi's Sarcoma, anti-HIV, anti-SIV and distinct hematopoietic effects of clinical grade crude preparations of human chorionic gonadotropin (hCG) (Lunardi-Iskandar et al. 1995, Gill et al. 1996, Lunardi-Iskandar et al. 1998). In contrast to their previous studies, it is also claimed that the antitumour and anti-viral activity of hCG preparation is not due to the native hCG heterodimer, including its purified subunits or its major degradation product, the $\beta$-core; instead the active moiety resides in an as yet unidentified hCG mediated factor (HAF) . Whatever the true factor may be, these unidentified factors in several hCG preparations have anti-tumour activity through the selective induction of apoptosis, besides direct cytotoxic effects on the tumour cells. Furthermore, they postulated that the anti-tumour activity could not be due to an immune-mediated response, since there was no infiltration of the tumour with mononuclear cells.

Moreover, the reported pro-hematopoietic effect of clinical grade hCG was noted in clinical studies in humans infected with HIV, (Lunardi-Iskandar et al. 1998) indicating that the hematopoietic effect is indirect, and caused by rescuing CD4+ cells otherwise killed by HIV through the anti-HIV activity of hCG.

Some auto-immune diseases, in particular Th1 mediated diseases, like rheumatoid arthritis (RA) (Grossman et al. 1997, Russel et al. 1997, Buyon et al. 1998, Hintzen et al. 1997) can remit during pregnancy. Furthermore, successful pregnancy is a Th2 type phenomenon (Raghupath et al. 1997) . We tested hCG preparation and its fractions from Pregnyl [Organon, Oss] on the development of diabetes in NOD mice and in a in vitro model.

Surprisingly, we found that intraperitoneal treatment of NOD mice of age 15 weeks, with a hCG preparation for three times a week for a month can delay or inhibit the onset of diabetes. In addition, transfer of total spleen cells from these treated NOD mice into NOD.scid mice can delay or prevent diabetes in NOD.scid whereas transfer of non-treated spleen cells cannot. This anti-diabetic effect resides in a fraction obtainable from pregnant woman but not in hCG.

Materials and methods

Mice. NOD mice were bred in our facilities under specific pathogen-free conditions. The spontaneous incidence of diabetes in our colony is 85% in females at 15 weeks of age. NOD.scid mice were also bred in our facilities under specific pathogen-free conditions. Transfer of diabetogenic cells from NOD to NOD.scid at the age of 8 weeks induces diabetes after 22 days. Diabetes. Diabetes was assessed by measurement of venous blood using an Abbott Medisense Precision Q.I.D. glucometer and also monitored for glucosuria (Gluketur Test; Boehringer Mannheim, Mannheim, Germany) . Animals were considered diabetic after two consecutive glucose measurements of higher than 13.75 mmol/l (250 mg/dl). Onset of diabetes was dated from the first consecutive reading. In instances of sustained hyperglycemia of >33 mmol/l animals were killed to avoid prolonged discomfort. Immunohistochemistry. Mice were killed by $CO_2$ asphyxiation. The entire pancreata were removed and snap frozen in OCT compound (Tissue-tek) for cry-sectioning. 5-$\mu$m cryo-sections were obtained, air dried, and stored at -20oC until used. Formalin-fixed sections were deparaffinized in xylene and alcohol, and stained with hematoxylin and eosin for general morphology.

Immunohistochemistry for insulin was then performed using a two-step protocol. Endogenous peroxidase activity was blocked, and slides were incubated with a rabbit antiserum to insulin (Dako Corp., Carpenteria, CA; 1:500 in 5% normal mouse serum for 30 min). After washing steps, staining was revealed with horseradish peroxidase-conjugated anti-rabbit Ig (Dako; 1:500 in 5% NMS for 30 min), developed with aminoethyl-carbazole (AEC; Pierce) for 10 min and mounted in crystalmount.

In vivo anti-diabetic effect: NOD mice at the age of 15 weeks were treated with PBS (n=4), 300 IU Pregnyl (n=4), or 600 IU Pregnyl (n=4) i.p., 3 times a week for four weeks and diabetes was assessed as mentioned above. After four weeks the treatment was stopped and the PBS and the 600 IU Pregnyl group were killed after one week. The 300 IU Pregnyl group was left alive till the age of 28 weeks. Spleen cell transfer. The spleen was removed from 600 IU Pregnyl treated NOD and PBS control treated NOD mice, and total spleen cells were recovered. These cells were washed twice with PBS and 20 x $10^6$ cells were i.p. transferred into a 8-wk-old NOD.scid mouse.

hCG preparation fractions and characterisation. Gel filtration of the solution of 1 or 2 vials of commercial grade hCG-Pregnyl (5,000 IU/vial) was performed on a Pharmacia FPLC sytem equipted with a Superdex 75 column (HR 5/30) (Pharmacia, Sweden) in PBS. Sample load volume was 1 ml. The flow rate was 0.5 ml/min for 45 min followed. The 1 minute flow rate of 0.2 ml/min was implemented because of the viscosity of the commercial grade hCG solution which has a high lactose content. hCG and a very low amount hCG core fragment were present in the relatively purified Pregnyl preparation of hCG and their positions were used as internal size markers. hCG eluted as 78kDa molecule and the hCG $\beta$-core eluted as a 19 kDa molecules on gel filtration. There were 1-5 fractions collected whereby fraction 1-2 contained hCG and fraction 5 contained the hCG (-core fragments. Fraction 1-2 and fraction 3-5 were tested for anti-diabetic effect by treating in vitro total spleen cells of 20-wk-old NOD and transferring them into NOD.scid. In this way human recombinant hCG, $\alpha$-hCG, and $\beta$-hCG (Sigma, St. Louis, MO. USA) were also tested.

In vitro restimulation. Total spleen cells (1 x $10^6$ cells/ml) from 20-wk-old NOD were stimulated in RPMI+ suplemented with 10% FBS with LPS (Ecoli;10 $\mu$g/ml) or coated anti-CD3 (145-2c11;25 $\mu$g/ml) with different doses of hCG-Pregnyl (50, 100, 300, 600, 800 IU/ml), Fraction 1-2 (200 $\mu$g/ml), Fraction 3-5 (200(g/ml), human recombinant hCG, $\alpha$-hCG, and $\beta$-hCG (each at 200 $\mu$g/ml) in flat bottom 96-well plates. Wells with anti-CD3 coating were implemented with IL-2 (40 IU/ml). Plates were incubated at 37°C in 5% CO2 in air for 48hrs. After 48hrs of incubation the supernatants were collected for cytokine analyses.

CD4+ T-cells were isolated from total spleen cells of 20-wk-old NOD and stimulated as mentioned above with anti-CD3 at different conditions. These wells were implemented with IL-2 (40 $\mu$g/ml) and anti-CD28 (10 $\mu$g/ml). After 48hrs of incubation the supernatants were also collected for cytokine analyses.

Cytokine ELISA. IL-4 was detected using monoclonal anti-IL-4 antibody (11B11) as the capture antibody and revealed with biotinated-conjugated rat anti-mouse IL-4 monoclonal ant-body (BVD6 24G2.3). IFN-$\gamma$ was detected using monoclonal anti-IFN-$\gamma$ antibody (XMG1.2) as the capture antibody and revealed with biotinylated-conjugated rat anti-mouse IFN-$\gamma$ monclonal antibody (R46A2). In both cases ABTS substrate was used for detection.

Results

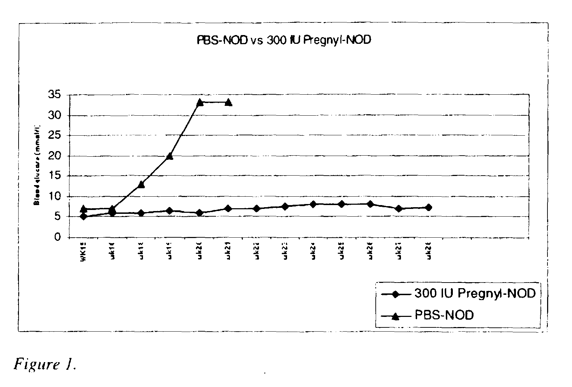

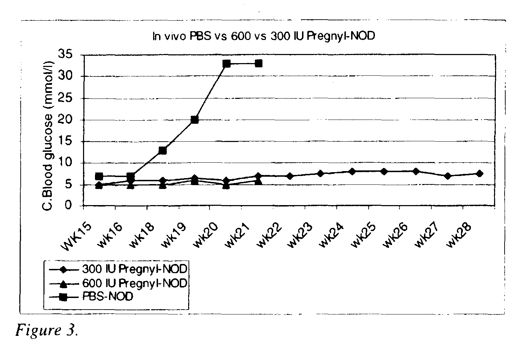

In vivo anti-diabetic effect: Four 15-wk-old NOD female mice (n=4) were treated with PBS, 300 IU Pregnyl, or 600 IU Pregnyl intraperitonealy, 3 times a week for four weeks. After the treatment all mice in the PBS group were diabetic (blood glucose >33 mmol/l), they lost weight and looked uncomfortable, while the 300 IU Pregnyl and 600 IU Pregnyl groups remained free of disease. Their blood glucose levels never exceeded 6 mmol/l and they looked very healthy (Figure 1 and 3). In order to assess possible infiltrations and intact insulin producing cells in the pancreas, mice from the PBS and the 600 IU Pregnyl groups were killed after treatment and entire pancreata were removed for immunohistochemistry for insulin. Pancreas sections from the PBS group showed many infiltrating cells in the pancreas and these cells penetrated the islets. There were also large number of B lymphocytes and T lymphocytes present in the pancreata of the PBS-group. This finding was consistent with our other finding of an elevated ratio of splenic CD8/CD4 cells due to a selective reduction in the number of CD4+ cells and a decrease in the number of B lymphocytes in the spleen of these mice (data not shown). In the 600 IU Pregnyl group, pancreata were free of infiltration and, surprisingly, a number of new insulin producing islets were seen. There was also a decrease in the number of B lymphocytes and T lymphocytes in pancreas, which was consistent with normal levels of the CD8/CD4 ratio and the number of B lymphocytes in the spleens of these mice. Mice from the 300 IU Pregnyl group were kept alive till the age of 28 weeks. They appeared healthy, did not loose their weight and never had blood glucose levels above 8 mmol/l (Figures 1 and 3). Immunohistochemistry for the presence of insulin was also performed. There were still infiltrating cells present and some insulin producing islets in the pancreas. These mice were treated for four weeks with Pregnyl along with the 600 IU Pregnyl group and from wk 20 till 28 they were left untreated.

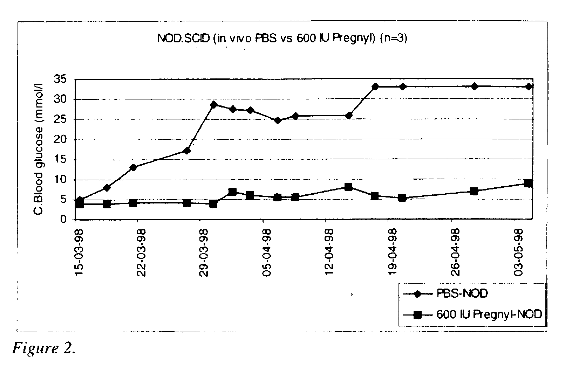

In order to determine whether the spleen cells of treated and untreated NOD mice still had the potential to induce diabetes in NOD.scid, we transferred spleen cells from the PBS and the 600 IU Pregnyl group into NOD.scid mice. 22 days after transferring, the PBS NOD.scid group were positive for diabetes and within a week they reached a blood glucose level above 33 mmol/l, while NOD.scid mice receiving spleen cells from the 600 IU Pregnyl group remained normal (blood glucose <7 mmol/l). 7 weeks after transferring, the PBS group looked very uncomfortable (Figure 2.), while the 600 IU Pregnyl. NOD.scid group still had blood glucose levels less than 9 mmol/l and remained healthy. Mice from both groups were killed at this time.

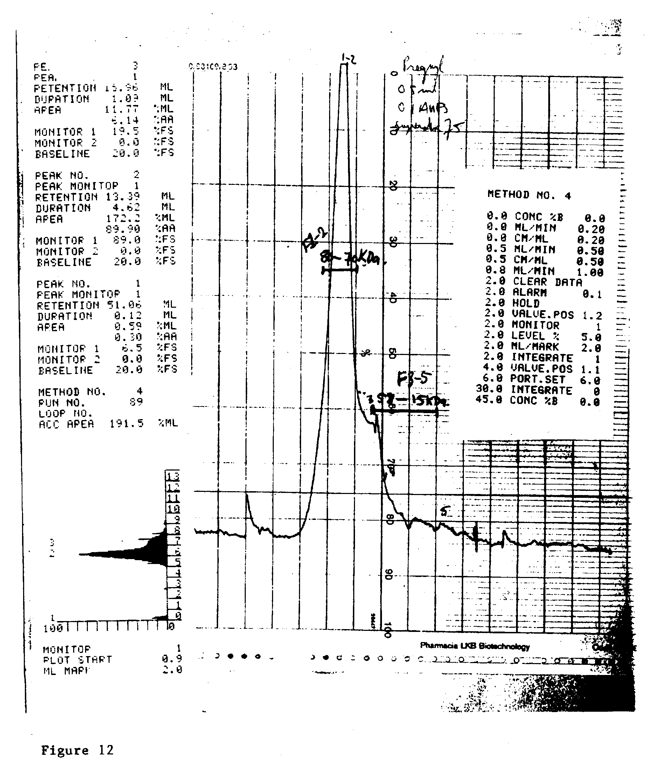

In vitro restimulation. Since high levels of IFN-$\gamma$, IL-1, and TNF-$\alpha$ were reported during the course of disease in NOD and this cytokine profile fits in a selective activation of the Th1 subset, we tested in vitro the effect of Pregnyl on cytokine production by total spleen cells and purified CD4+ cells from 20-wk-old NOD female mice. In order to assess whether the anti-diabetic effect resides in hCG or in one of its subunits or in other factors contained in the preparation used , we also tested the effect of different fractions obtained by gelpermeation chromatography from Pregnyl (Figure 12) and human recombinant hCG and its subunits on cytokine production. The effect of these fractions were also tested in vivo on blood glucose levels in reconstituted NOD.scid mice.

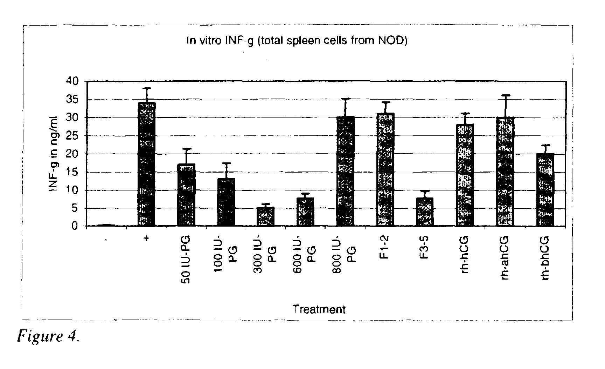

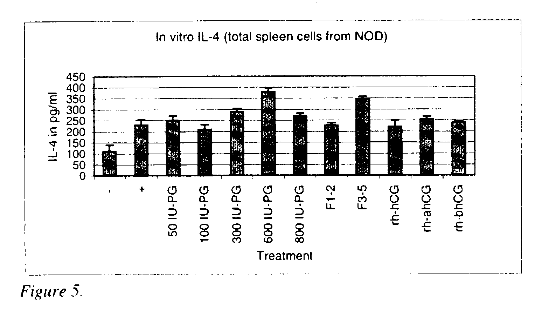

We observed a strong inhibition of IFN-$\gamma$ production by spleen cells obtained from mice treated with 50-600 IU/ml of Pregnyl, F3-5 (58-15 Kda) and to a lesser extent with human recombinant-$\beta$CG (Figures 4-6). There was only a moderate increase in IFN-$\gamma$ production splenocytes from mice treated with 800 IU/ml Pregnyl. A similar pattern was observed when analyzing IL-4 production (Figure 5). In addition a marked inhibition of IL-1 and TNF-$\alpha$ production was observed in stimulated splenocytes from mice treated with 300-600 IU/ml Pregnyl, with a concomitant stimulation of IL-6 and IL-10 production (data not shown).

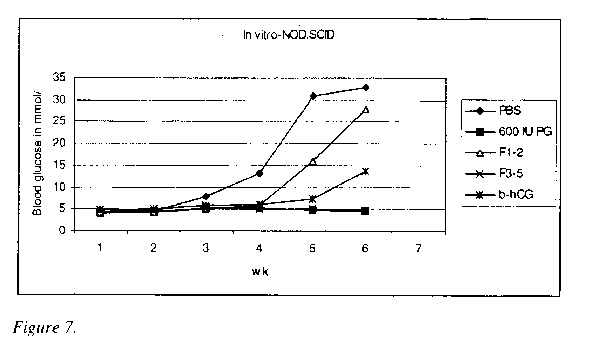

Furthermore, transfer experiments showed that total spleen cells of 20-wk-old NOD mice treated with F3-5 or 600 IU Pregnyl can delay or even prevent the onset of diabetes in NOD.scid as compared to reconstitution with PBS treated NOD cells (Figure 7). However, no significant effect was observed with F1-2 (80-70 Kda) on the onset of diabetes in NOD.scid mice. In order to test whether Pregnyl has also effect on Th2 type mice, we treated BALB/c mice (n=5) with 300 IU Pregnyl i.p. for four days and with PBS (n=5). After isolating CD4+ cells from spleens we stimulated them with anti-CD3/IL-2 for 48 hours and the supernatants were collected for the determination of IFN-$\gamma$ and IL-4 cytokines. We also treated CD4+ cells with different doses of Pregnyl. Subsequently the supernatants were collected for cytokine anaylses. There was a marked inhibition of IFN-$\gamma$ and a concomitant stimulation of IL-4 found in CD4+ cells stimulated with anti-CD3/IL-2 only (Th1->Th2), while the inverse was seen in CD4+ cells treated in vitro with different doses of Pregnyl (Th2->Th1).

Discussion

Nonobese diabetic (NOD) mice naturally develop an insulin-dependent diabetes (IDDM) with remarkable similarity in immunopathology and clinical symptoms to human IDDM patients. As a result, NOD mice have become a valuable tool for studying the underlying immunobiology of IDDM and the complex genetics that control it. Through their study we now know that diabetes is caused by a disbalance in the ratio of the Th1/Th2 subsets and consequently, the destruction of insulin producing $\beta$-cells. This destruction is coordinated by $\beta$-cell antigen-specific CD4+ T cells that produce proinflammatory cytokines like IFN-$\gamma$, TNF-$\alpha$/$\beta$, and IL-1. A growing number of studies has now correlated diabetes (in mice and in humans) with a preferential development of Th1-like cells.

In contrast, pregnancy is thought to be a selective Th2 phenomenon, and surprisingly during pregnancy the severity of many immune-mediated diseases has been seen reducing. In contrast, Gallo et al. have shown that hCG mediated factor(s) (HAF) present in the urine of first trimester pregnancy have an anti-tumour (and anti-viral) effect, which is possibly achieved by a direct cytotoxic effect on tumour cells and, according to these authors, not by an immune-mediated response.

Here we show an immunoregulator obtainable for example from urine of (first trimester) pregnancy not only effects the above mentioned immune deviation during pregnancy, but also effects the development of diabetes in NOD mice.

Our results show that for example Pregnyl, a partially purified hCG preparation from urine of first trimester pregnancy, can delay the onset of diabetes, for example in 15-wk-old NOD when treated only for 3 times a week during four weeks. In addition, spleen cells isolated from these treated mice upon transfer have also the potential to delay the onset of diabetes in immuno-compromised NOD.scid mice. We fractionated a Pregnyl preparation to assess whether this anti-diabetic activity resides in hCG itself, its subunits, β-core (naturally break-down product of β-hCG) or in unidentified factors (HAF). It is worth knowing that Pregnyl is one of the most purified hCG preparations available and it contains only low amounts of β-core fragments. We found that most of the anti-diabetic activity resided in a fraction without hCG. Furthermore, we showed that human recombinant α-hCG and β-hCG also had no effect. However, we do not exclude the possibility that hCG can synergize with other factors in diabetes and other immune mediated diseases.

Immunohistological analysis of the presence of insulin and infiltration in the pancreas of NOD mice showed that NOD mice treated with 600 IU Pregnyl did not reveal a significant infiltrate. Moreover, new insulin islets were seen the in pancreas, which shows a possible regeneration process induced by this treatment. As mentioned before, normally at the age of 9 weeks infiltrating cells penetrate into the islets and the islets become swollen with lymphocytes. In our experiments, the NOD mice were 15-wk-old and the PBS treated control mice had many infiltrating cells and almost no insulin producing cells at that time in their pancreas. In addition, PBS treated mice had also an elevated ratio of CD8/CD4 in their spleen and many T cells in their pancreas. Since our treated mice had a normal CD8/CD4 ratio in their spleen and no infiltration was found in their pancreas, the elevated CD8/CD4 ratio was due to selective recruitment of CD4+ cells into the pancreas. IFN-γ and TNF-α are involved in the recruitment of T lymphocytes (Rosenberg at al. 1998).

Our results show that treatment of NOD mice with 600 IU Pregnyl for four weeks had dramatic effects on the morphology and function of their otherwise inflamed pancreas. Furthermore, our 300 IU Pregnyl NOD mice were kept alive till the age of 28 weeks without treatment and remained non-diabetic. The 600 IU Pregnyl NOD mice were also examined for symptoms of generalised auto-immune diseases, like Sjögren's disease, which were not found.

Our in vitro experiments with total spleen cells and purified CD4+ cells of NOD are consistent with the in vivo data. There was marked inhibition of IFN-γ, IL-1 and TNF-α release by spleen cells (data not shown) from NOD mice treated in vitro with Pregnyl, F3-5, and to lesser extent with human recombinant β-hCG. Increase in IL-4 production was also observed implying a shift of Th1 to Th2 type response with the treatment. However, doses above 800 IU Pregnyl caused opposite results and may be due to the presence of high amount of hCG itself.

The immune system is clearly involved in the onset of diabetes. Treatment with Pregnyl effects the immune system and thereby can reduce the disease activity in NOD mice. In order to separate the immune-modulating activity of Pregnyl from its benefical clinical effect, we treated healthy BALB/c mice. This strain is generally considered to react upon stimulation with a Th2 driven immune response. Our results suggest that purified CD4+ T cells obtained from Pregnyl-treated BALB/c mice display a further Th2 skewing. The same cells when restimulated with Pregnyl in vitro showed an enhancement of IFN-γ production and a decrease in IL-4 production. This implies that Pregnyl effects different regulatory T cells subsets upon treatment in vivo versus in vitro. We suggest that treatment in vivo stimulates the outgrowth of a population of presumably CD4+ Tr1 cells, characterised by selective production of TGF-β and a lower or no production of IL-10. These CD4+ Tr1 cells have been shown (O'Garra et al. 1997) in different models of Th1 driven diseases including diabetes and MS, to selectively inhibit the activity of Th1 cells, thereby decreasing the disease severity also. Similar by CD4+ T cells from Pregnyl treated BALB/c mice restimulated in vitro with Pregnyl showed an increase of Th1 cells concomitant with a decrease of Th2 cells. This is consistent with a preferential stimulation of the CD4+ Th3 cells characterized by a high production of IL-10 and a low production of TGF-β. These regulatory cells are inhibitors of IFN-γ production by Th1 cells as well as the outgrowth of Th2 type cells. It has been also shown that in NOD.scid mice a steady increase of Th2 cells is responsible for the less severe hyperglycemia and the different nature of the infiltrates in the pancreatic islets.

Our results of the 300 IU Pregnyl treated NOD and our reconsituted NOD.scid mice showed a similar slow increase in blood glucose, particularly in NOD.scid, and a different nature of the infiltrates as compared to PBS-treated NOD. In NOD mice the activity of Pregnyl might well be mediated with the induction of Th3 cells inhibiting both Th1 and Th2 cells. These Th3 cells may suppress the disease activity for prolonged periods of time at the very least. In NOD.scid mice, having no functional T cells, reconstitution with Pregnyl-treated spleen cells is mediated with selective induction of Tr1 cells and thereby inhibiting the Th1 subset only. After prolonged periods the steady outgrowth of diabetogenic Th2 cells is responsible for the late onset of a less severe form of diabetes. Similarly our F3-5, but not F1-2, displays the above discussed phenomenon, arguing that hCG can not be responsible for the observed effects. This F3-5 is principally pointing towards a decisive effect on the immune response in the onset of auto-immune diabetes and is an active component for immunotherapy of this disease and other immune mediated disorders.

In addition, Pregnyl and immunoregulators functionally equivalent thereto, is effective in Non-insulin-diabetes mellitus (NIDDM) . The essential problem in NIDDM patients is the insulin resistancy and obesity, it has been shown that TNF-(alpha) is the cause of the insulin resistance of obesity and NIDDM (Miles et al. 1997, Solomon et al. 1997, Pfeiffer et al. 1997, Hotamisligil et al. 1994), Argiles et al. 1994). This insulin resistance induced by TNF-alpha can be reversed by recently developed medicines like Pioglitazone and Metformin, and with engineered human anti-TNF-alpha antibody (CDP571) (Solomon et al. 1997, Ofei et al. 1996), which possibly achieved their benefical action by lowering TNF-alpha induced free fatty acids (FFA) concentration of the blood and/or by stimulating glucose uptake at an intracellular point distal to insulin receptor autophosphorylation in muscle. Furthermore, the presence of retinopathy (Pfeiffer et al. 1997) (one of the late complications of diabetes) has been mediated with significantly elevated plasma TNF-alpha and is sex-dependent (Pfeiffer et al. 1997). The increased TNF-alpha occurs in male but not in female NIDDM and may participate in the development of retinopathy and other complications like neuropathy, nephropathy or macroangiopathy (Pfeiffer et al. 1997). Since Pregnyl and fraction 3-5 have immune modulating potential and in particular inhibit TNF-alpha directly or indirectly, Pregnyl and its fraction 3-5 have also benefical effects in NIDDM patients. Besides, lower incidence of diabetes complications among female could implicate the involvement of female hormones.

Thus, Pregnyl and its fraction 3-5 have high potency to suppress auto-immune diabetes by modulating the immune system by effecting regulatory T cells subsets. Our NOD and BALB/c data show that they have the potential to restore the T-cell subset balance (Th1->Th2/Th2->Th1). Therefore, Pregnyl and its fraction 3-5 are effective in modulating the severity of other immune-mediated diseases too, like diseases where Th1 cytokines are dominant such as Rheumatoid Arthritis (RA), Multiple Sclerosis (MS), NIDDM, Systemic lupus erythematosus (SLE), transplantation models and diseases like allergies and asthma where Th2 cytokines responses are dominant. Animal models of these diseases (like EAE-model for MS, BB-rats for NIDDM, Fishe-rat and MLR-models for RA, OVA-model for allergies, MLR/lpr and BXSB-models for SLE), KK-Ay-mice, GK rats, wistar fatty rats, and fa/fa rats provide, amongst others, models of other immune-mediated or mediated diseases.

Figures

Figure 1. Shows that 15-weeks-old NOD mice treated with PBS for 4 weeks, become diabetic (>13.75 mmol/l) at the age of 17 weeks and within a week they had blood glucose levels above 30 mmol/l, while NOD mice treated with 300 Pregnyl remained nondiabetic till they were killed (at the age of 28-weeks) even the treatment was stopped at age 19 weeks. There blood glucose level remained lower than 8 mmol/l.

Figure 2. shows that reconsituted NOD.scid mice receiving spleen cells from PBS treated NOD mice(fig.3) became diabetic after 22 days of transferring, while reconsituted NOD.scid mice with 600 IU Pregnyl treated NOD remained nondiabetic till they were killed (8 weeks after transferring).

Figure 3. Shows that 15-weeks-old NOD mice treated with PBS for 4 weeks, become diabetic (>13.75 mmol/l) at the age of 17 weeks and within a week they had blood glucose levels above 30 mmol/l, while NOD mice treated with 600 IU Pregnyl remained nondiabetic till they were killed along with PBS group (at the age of 21-weeks). 15-weeks-old NOD mice treated with 300 IU Pregnyl remained nondiabetic till they were killed (at the age of 28-weeks) even the treatment was stopped at age 19 weeks. There blood glucose levels remained lower than 8 mmol/l.

Figure 4. Spleens cells from 20-weeks-old female NOD were islolated and were cultured for 48hrs with different conditions ('-' only medium, '+' with anti-CD3,50, 100, 300, 600, 800 IU/ml Pregnyl, F1-2, F3-5, rh-hCG, rh-alpha-hCG, rh-beta-hCG [each at 200ug/ml]) in the presence of anti-CD3 and IL-2. After 48hrs INF-( cytokine ELISA were done. Results shows that there is dose dependent inhibition of INF-( with Pregnyl (50-600 IU/ml) and fraction 3-5 (F3-5) containing no hCG. There is an increase in INF-g with 800 IU/ml Pregnyl which suggests the effect of hCG itself. NO effect on INF-g were seen with fraction 1-2 (F1-2) containing hCG, human recombinant(rh) hCG, rh-alpha-hCG. Slight decrease in INF-g level is seen with rh-beta-hCG.

Figure 5. Spleens cells from 20-weeks-old female NOD were isolated and were cultured for 48hrs with different conditions ('-' only medium, '+' with anti-CD3,50, 100, 300, 600, 800 IU/ml Pregnyl, F1-2, F3-5, rh-hCG, rh-alpha-hCG, rh-beta-hCG [each at 200$\mu$g/ml]) in the presence of anti-CD3 and IL-2. After 48hrs IL-4 cytokine ELISA was done. Results shows that there is a dose dependent increase of IL-4 with Pregnyl (50-600 IU/ml) and fraction 3-5 (F3-5) containing no hCG. There is an decrease in IL-4 with 800 IU/ml Pregnyl which suggests the effect of hCG itself. NO effect on IL-4 were seen with fraction 1-2 (F1-2) containing hCG, human recombinant(rh) hCG, rh-alpha-hCG and rh-beta-hCG.

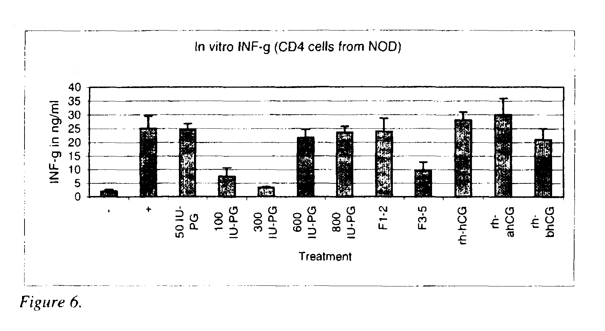

Figure 6. CD4 T-cells from spleen of 20-weeks-old female NOD were islolated and were cultured for 48hrs with different conditions ('-' only medium, '+' with anti-CD3,50, 100, 300, 600, 800 IU/ml Pregnyl, F1-2, F3-5, rh-hCG, rh-alpha-hCG, rh-beta-hCG [each at 200μg/ml]) in the presence of anti-CD3, IL-2 and anti-CD28. After 48hrs INF-γ cytokine ELISA were done. Results shows that there is dose dependent inhibition of INF-γ with Pregnyl (50-300 IU/ml) and fraction 3-5 (F3-5) containing no hCG. There is an increase in INF-γ with 600- 800 IU/ml Pregnyl which suggests the effect of hCG itself. NO effect on INF-g were seen with fraction 1-2 (F1-2) containing hCG, human recombinant(rh) hCG, rh-alpha-hCG. Slightly decrease in INF-γ level is seen with rh-beta-hCG.

Figure 7. Show the transfer experiment of 20-weeks old female spleen cells treated with PBS, 600 IU Pregnyl, fraction 1-2(F1-2), Fraction 3-5(F3-5) or human recombinant beta-hCG (b-hCG) for 48hrs and then transferred into 8-weeks old NOD.scid (n=3). After 22 days of transfer the NOD.scid mice receiving PBS treated NOD spleens were diabetic. NOD.scid mice receiving F1-2 and b-hCG were diabetic after 4 and 5 weeks respectively while NOD.scid mice receiving 600 IU Pregnyl and F3-5 remained nondiabetic about 6 weeks and then all mice were killed. It shows that the maximum antidiabetic effect resides in Pregnyl and F3-5. Since F1-2 which contain mostly hCG have no effect on the incidence of diabetes in these mice, it is clear that antidiabetic effect does not reside in hCG itself. There is slightly anti-diabetic affect in recombinant human beta-hCG.

Figures 8-11

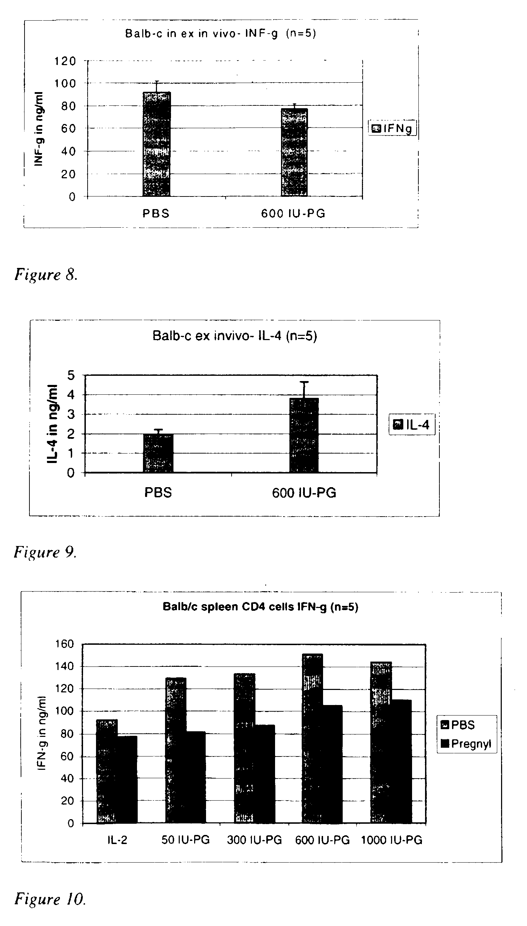

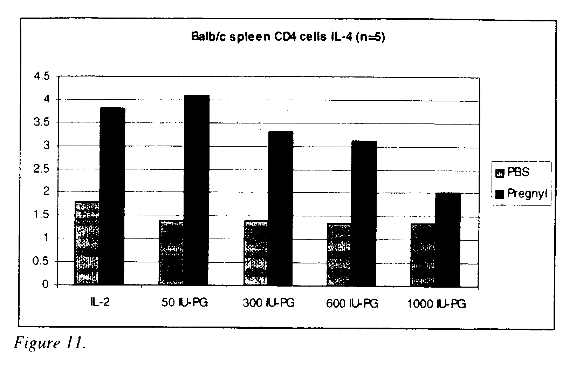

In order to test whether Pregnyl has also effect on Th2 type mice, we treated BALB/c mice (n=5) with 300 IU Pregnyl i.p. for four days and with PBS (n=5). After isolating CD4+ cells from spleens we stimulated them with anti-CD3/IL-2 for 48 hours and the supernatants were collected for the determination of IFN-γ (figure 8) and IL-4 (figure 9) cytokines. We also treated CD4+ cells with different doses of Pregnyl. Subsequently the supernatants were collected for INF-g ELISA (Figure 10) analyses. Figure 8 shows the invivo treatment with 300 IU Pregnyl suppress INF-g and on the other hand increases IL-4 production. This implies that there is more shift towards Th-2 phenotype. Same cells treated again in vitro with different dosis of Pregnyl show (Figure 10) increase in INF-g and decrease in IL-4 (figure 11) which suggest the shift towards Th-1 phenotype. This all implies that Pregnyl and F3-5 have affect on regulatory T-cell subset (Th3, Tr1).

Figure 12

column

Superdex 75 HR 10/30; FPLC system (Pharamcia) total volume $V_t$= 25 ml; void volume $V_0$=8.7ml; flow rate: 1 ml/min; buffer: 10mM phosphate-buffered saline, pH 7.3; at room temperature
column efficiency=38,000 N/m
selectivity $K_{AV}$ = 1.737 - 0.2782 log ($r^2$ = 0.982), MW = molecular mass
separation range: 3,000 - 100,000 Dalton for globular proteins

running method

| METHOD NO. 4 | | |
|---|---|---|
| 0.0 | CONC%B | 0.0 |
| 0.0 | ML/MIN | 0.20 |
| 0.0 | CM/ML | 0.20 |
| 0.5 | ML/MIN | 0.50 |
| 0.5 | CM/ML | 0.50 |
| 0.8 | ML/MIN | 1.00 |
| 2.0 | CLEAR DATA | |
| 2.0 | ALARM | 0.1 |
| 2.0 | HOLD | |
| 2.0 | VALVE.POS | 1.2 |
| 2.0 | MONITOR | 1 |
| 2.0 | LEVEL % | 5.0 |
| 2.0 | ML/MARK | 2.0 |
| 2.0 | INTEGRATE | 1 |
| 4.0 | VALVE.POS | 1.1 |
| 6.0 | PORT.SET | 6.0 |
| 30.0 | INTEGRATE | 0 |
| 45.0 | CONC %B | 0.0 |

sample

Pregnyl (Organon, lot nr.:168558, exp.date:28.11.99)
sample volume = 0.5 ml = 2,000 units; sensitivity 0.1 AUFS

chromatogram

Peak 1 = fractions 1-2: Ve = 14.7 - 15.1 ml;$K_{AV}$=0.37-0.39
Peak 2 = fractions 3-5: Ve = 15.38 - 17.99 ml;
$K_{AV}$ = 0.41 - 0.57
$$K_{AV} = (V_e - V_0)/(V_t - V_0)$$

Peak 1 elutes at a volume between 14.7 - 15.1 ml after start of the separation. This corresponds to a molecular mass between 70,000 - 80,000 Dalton. This fraction contains in part the dimeric form of hCG (Textbook of Endocrine Physiology, Second edition, J.E. Griffin, S.R. Ojeda (Ed.) Oxford University Press, Oxford, 1992, pp.199). Peak 2 elutes at a volume between 15.38 and 17.99 ml, corresponding to a volume between 1500 - 58,000 Dalton. This fraction contains partly β-subunit (MW=22,200 Dalton), breakdown products of hCG and other, as yet, unknown molecules. These calculations were based on the above-mentioned selectivity of this column.

References

Abbas, A.K., K.M. Murphy, and A. Sher. 1996. Functional diversity of helper T lymphocytes. Nature (Lond.). 383:787-793.

Argues J.M., Lopez-Soriano J., Lopez-Soriano F.J. 1994. Cytokines and diabetes: the final step? Involvement of TNF-alpha in both type I and II diabetes mellitus. Horm. Metab.Res. 26:447-449.

Atkinson, M.A., and N.K. Maclaren. 1994. The Pathogenesis of insulin-dependent diabetes mellitus. N. Engl. J. Med. 331: 1428-1436.

Bach, J.F. 1991. Insulin-dependent diabetes mellitus. Curr. Opin. Immunol. 3:902-905.

Buyon J.P. 1998. The effcts of pregnancy on autoimmune diseases. J. Leukoc. Biol. 63:281-287.

Elias D. 1994. The NOD mouse: a model for autoimmune insulin-dependent diabetes. (book) Autoimmune Models: A guidebook. 147-161.

Gill, P.S., Lunardi-Iskandar, Y.Gallo R.C. 1996. The effects of preparations of human chorionic gonadotropin on AIDS-related kaosi's Sarcoma. New Eng. J. Med. 335:1261-1269.

Grossman J.M., Brahn E. 1997. Rheumatoid arthritis: current clinical and research directions. J. Womens Health.6:627-638.

Harada, M., and S. Makino. 1986. Suppression of overt diabetes in NOD mice by anti-thmocyte serum or anti-Thy 1, 2 antibody. Jikken. Dobutsu. 35:501-504.

Hintzen R.Q. 1997. Th-cell modulation in multiple sclerosis. Immunology today. 18:507-508.

Hotamisligil G.S., Spiegelman B.M. 1994. Tumour necrosis factor alpha: a key component of the obesity-diabetes link. Diabetes 43:1271-1278.

Katz, J.D., C. Benoist and D. Mathis. 1995. T helper cell subsets in insulin-dependent diabetes. Science (Wash. DC). 268:1185-1188.

Liblau, R.S., S.M. Singer, and H.O. McDevitt. 1995. Th1 and Th2 CD4+ T cells in the pathogenesis of organ-specific auto-immune diseases. Immunol. Today. 16:34-38. Lunardi-Iskandar, ...Gallo R.C. 1995. Tumourigenesis and metastasis of neoplastic kaposi's sarcoma cell line in immunodeficient mice blocked by a human pregnancy hormone. Nature (Lond.). 375:64-68.

Lunardi-Iskandar, Y., Bryant, J.L. Blattner W.A., Hung C.L, Flamand L, Gill P., Hermans P., Birken S., and Gallo R.C. 1998. Effects of a urinary factor from women in early pregnancy on HIV-1, SIV and mediated disease. Nature Med. 4:428-434.

Makino, S., M. Harada, Y. Kishimoto, and Y. Hayashi. 1986. Absence of insulitis and overt diabetes in athymic nude mice with NOD genetic background. Jikken. Dobutsu. 35:495-498.

Miles P.D., Romeo O.M., Higo K., Cohen A., Rafaat K., Olefsky J.M. 1997.TNF-alpha-induced insulin resistance in vivo and its prevention by troglitazone. Diabetes 46:1678-1683.

Miyazaki, A., T. Hanafusa, K. Yamada, J. Miyagawa, H. Fujino-Kurihara, H. Nakajima, K. Nonaka, and S. Tarui. 1985. Predominance of T lymphocytes in pancreatic islets and spleen of pre-diabetic non-obese diabetic (NOD) mice: a longitudinal study. Clin. Exp. Immunol. 60:622-630. Ofei F., Hurel S., Newkirk J., Sopwith M., Taylor R. 1996. Effects of an engineered human anti-TNF-alpha antibody (CDP571) on insulin sensitivity and glycemic control in patients with NIDDM. Diabetes 45:881-885. O'Garra, A., Steinman, L, and Gijbels, K. 1997. CD4+ T-cell subsets in auto-immunity. Curr. Opin. Immunol. 9(6):872-883.

O'Reilly, L.A., P.R. Hutchings, P.R. Crocker, E. Simpson, T. Lund, D. Kiossis, F. Takei, J. Baird, and A. Cooke. 1991. Characterization of pancreatic islet cell infiltrates in NOD mice: effect of cell transfer and transgene expression. Eur. J. Immunol. 21:1171-1180. Pakala, S.V., Kurrer, M.O. and Katz, J.D. 1997. T helper 2 (Th2) T cells induce acute pancreatitis and diabetes in immune-compromised nonobese diabetic (NOD) mice. J. Exp. Med.

186:299-306.

Pfeiffer A., Janott J., Mohlig M., Ristow M., Rochlitz H., Busch K., Schatz H., Schifferdecker E. 1997. Circulating tumour necrosis factor alpha is elevated in male but not in female patients with type II diabetes mellitus. Horm. Metab Res. 29:111-114.

Raghupathy R. 1997. Th1-type immunity is incompatible with successful pregnancy. Immunology today. 18:478-482. Rosenberg, Y.J., Anderson, A.O., and Pabst, R. 1998. HIV-induced decline in blood CD4/CD8 ratios: viral killing or altered lymphocyte trafficking?. Immunology Today 19:10-16.

Russell A.S., Johnston C., Chew C., Maksymowych W.P. 1997. Evidence for reduced Th1 function in normal pregnancy:a hypothesis for the remission of rheumatoid arthritis. J. Rheumatol. 24:1045-1050.

Sempe, P., P. Bedossa, M.F. Richard, M.C. Villa, J.F. Bach, and C. Boitard. 1991. Anti-alpha/beta T cell receptor monoclonal antibody provides an efficient therapy for auto-immune diabetes in nonobese diabetic (NOD) mice. Eur. J. Immunol. 21:1163-1169.

Solomon S.S., Mishra S.K., Cwik C., Rajanna B., Postlethwaite A.E. 1997. Pioglitazone and metformin reverse insulin resistance induced by tumour necrosis factor-alpha in liver cells. Horm. Metab. Res. 29:379-382.

Zhu X.P, Satob J., Muto G., Muto Y., Sagara M., Takahashi K., Seino H., Hirai S., Masuda T., Tanaka S., Ishida H.,

Seino Y., Toyota T. 1996. Improvement of glucose tolerance with immunomodulators on type 2 diabetic animals. Biotherapy 9:189-197.

**Claims**

1. An immunoregulator comprising an active component obtainable from a mammalian chorionic gonadotropin preparation present in a fraction which elutes with an apparent molecular weight of 58 to 15 kilodalton as determined in gel-permeation chromatography, said active component capable of stimulating splenocytes obtained from a non-obese diabetes (NOD) mouse, or comprising an active component functionally related to said active compound.

2. An immunoregulator according to claim 1 wherein said stimulated splenocytes are capable of delaying the onset of diabetes in a NOD-severe-combined-immunodeficient mouse reconstituted with said splenocytes.

3. An immunoregulator according to claim 1 or 2 wherein said active component is capable of inhibiting gamma-interferon production of splenocytes obtained from a nonobese diabetes (NOD) mouse.

4. An immunoregulator according to anyone of claims 1 to 3 wherein said active component is capable of stimulating interleukine-4 production of splenocytes obtained from a non-obese diabetes (NOD) mouse.

5. Use of an immunoregulator according to anyone of claims 1-4 for the production of a pharmaceutical composition for the treatment of an immune-mediated-disorder.

6. Use according to claim 5 wherein said immunoregulator comprises a hCG preparation or a fraction derived thereof.

7. Use according to claim 5 or 6 wherein said disorder comprises diabetes.

8. A pharmaceutical composition for treating an immune-mediated disorder comprising an active component having an apparent molecular weight of 58 to 15 kilodalton as obtainable by gel-permeation chromatography, said active component capable of stimulating splenocytes obtained from a non-obese diabetes (NOD) mouse, said stimulated splenocytes delaying the onset of diabetes in a NOD-severe-combined-immnunodeficient mouse reconstituted with said splenocytes, or comprising an active component functionally related to said active component.

9. A pharmaceutical composition for treating an immune-mediated disorder according to claim 8 wherein said active component is capable of inhibiting gamma-interferon production or stimulating interleukine-4 production of splenocytes obtained from non-obese diabetes (NOD) mouse.

10. A pharmaceutical composition for treating an immune-mediated disorder according to claim 8 or 9 obtainable from a pregnant mammal, preferably a human.

11. A pharmaceutical composition for treating an immune-mediated disorder according to claim 10 obtainable from a clinical grade hCG preparation or a fraction derived thereof.

12. A method for treating an immune-mediated-disorder comprising subjecting an animal to treatment with at least one immunoregulator according to any one of claims 1 to 4.

13. A method according to claim 12 wherein said disorder comprises diabetes.

14. A method according to any one of claims 12 to 13 further comprising regulating relative ratios and /or cytokine activity of lymphocyte subset-populations in said animal.

15. A method according to claim 14 wherein said subset-populations comprise Th1 or Th2 cells.

*Figure 1.*

*Figure 2.*

Figure 3.

*Figure 4.*

*Figure 5.*

*Figure 6.*

*Figure 7.*

*Figure 8.*

*Figure 9.*

*Figure 10.*

*Figure 11.*

```
PE.            3        0.03100,203
PEA.           1
RETENTION  15.96   ML
DURATION    1.09   ML
APEA       11.77   %ML
            5.14   %AA
MONITOR 1  19.5    %FS
MONITOR 2   0.0    %FS
BASELINE   20.0    %FS

PEAK NO.       2
PEAK MONITOR   1
RETENTION  13.39   ML
DURATION    4.62   ML
APEA      172.2    %ML
           89.90   %AA
MONITOR 1  89.0    %FS
MONITOR 2   0.0    %FS
BASELINE   20.0    %FS

PEAK NO.       1
PEAK MONITOR   1
RETENTION  51.06   ML
DURATION    0.12   ML
APEA        0.59   %ML
            0.30   %AA
MONITOR 1   6.5    %FS
MONITOR 2   0.0    %FS
BASELINE   20.0    %FS

METHOD NO.     4
PUN NO.       89
LOOP NO.
ACC APEA  191.5   %ML
```

```
METHOD NO.  4

0.0  CONC %B     0.0
0.0  ML/MIN      0.20
0.0  CM/ML       0.20
0.5  ML/MIN      0.50
0.5  CM/ML       0.50
0.8  ML/MIN      1.00
2.0  CLEAR DATA
2.0  ALARM       0.1
2.0  HOLD
2.0  VALUE.POS  1.2
2.0  MONITOR      1
2.0  LEVEL %      5.0
2.0  ML/MARK      2.0
2.0  INTEGRATE    1
4.0  VALUE.POS  1.1
6.0  PORT.SET    6.0
30.0 INTEGRATE    0
45.0 CONC %B      0.0
```

```
MONITOR        1
PLOT START    0.9
ML MARK       2.0
```

Pharmacia LKB Biotechnology

Figure 12

European Patent
Office

**PARTIAL EUROPEAN SEARCH REPORT**

**Application Number**

which under Rule 45 of the European Patent Convention
shall be considered, for the purposes of subsequent
proceedings, as the European search report

EP 98 20 1695

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | LUNARDI-ISKANDAR ET AL: "Effects of a urinary factor from women in early pregnancy on HIV-1, SIV and associated diseases" NATURE MEDICINE, vol. 4, no. 4, April 1998, pages 428-434, XP002080995 * the whole document * | 1-6,8-12 | A61K38/17 A61K38/24 |
| D,A | LANG ET AL: "Induction of apoptosis in Kaposi's sarcoma spindle cell cultures by the subunits of human chorionic gonadotropin" AIDS, vol. 11, 1997, pages 1333-1340, XP002080996 * page 1333 * * abstract * | 1-15 | |

TECHNICAL FIELDS
SEARCHED       (Int.Cl.6)

A61K

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

Although claims 12-15 are directed to a method of
treatment of the human/animal body (Article 52(4)
EPC), the search has been carried out and based on the
alleged effects of the compound/composition.

| Place of search | Date of completion of the search | Examiner |
|---|---|---|
| THE HAGUE | 15 October 1998 | Sitch, W |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons

& : member of the same patent family, corresponding
      document